# FASCICULE DE BREVET EUROPEEN

(11) **EP 2 783 056 B1**
(45) Date de publication et mention de la délivrance du brevet: **20.09.2017**
(21) Numéro de dépôt: 12790537.0
(22) Date de dépôt: 20.11.2012
(51) Int. Cl.: E04H 4/16, G01N 33/18, G08B 21/08

(54) **SYSTÈME DE SURVEILLANCE D'UN BASSIN ET PROCÉDÉ DE SURVEILLANCE ASSOCIÉ**
BECKENÜBERWACHUNGSSYSTEM UND ENTSPRECHENDES ÜBERWACHUNGSVERFAHREN
POOL SURVEILLANCE SYSTEM AND ASSOCIATED SURVEILLANCE METHOD

(30) Priorité: 21.11.2011 FR 1160588
(43) Date de publication de la demande: 01.10.2014
(60) Demande divisionnaire: 15150537.7
(73) Titulaire: Softbank Robotics Europe, 75015 Paris (FR)
(72) Inventeur: MAISONNIER, Bruno, F-75014 Paris (FR); BARDINET, Fabien, F-92130 Issy Les Moulineaux (FR)
(74) Mandataire: Collet, Alain
(86) Numéro de dépôt international: PCT/EP2012/073102
(87) Numéro de publication internationale: WO 2013/076076

(56) Documents cités:
- EP-A1- 1 492 069
- WO-A1-99/56214
- WO-A1-2004/019295
- WO-A1-2006/027456
- WO-A1-2011/003923
- US-A1- 2008 106 422

## Description

Le domaine de l'invention est celui des systèmes de surveillance de bassins, tels que des piscines, comprenant un capteur apte à produire une mesure représentative d'une perturbation du bassin par rapport à un état de référence.

Plus particulièrement, le domaine de l'invention est celui des dispositifs de surveillance de la qualité de l'eau stockée dans les bassins. Le domaine de l'invention est également celui des systèmes de surveillance de la survenue d'une intrusion d'un individu dans le bassin.

Les bassins, tels que les piscines privées ou publiques, sont remplis d'un liquide tel que de l'eau. Le renouvellement constant de l'eau est inenvisageable. Toutefois, la composition de l'eau n'est pas stable et la qualité de l'eau est susceptible de se dégrader. Or, il est nécessaire d'assurer un certain confort d'utilisation ainsi que de respecter des conditions de sécurité prédéterminées. Il est donc courant d'équiper de tels bassins d'un système de surveillance de la qualité de l'eau. Un tel système comprend des capteurs susceptibles de mesurer des grandeurs physico-chimiques représentatives de la qualité de l'eau. Les capteurs sont, par exemple, aptes à mesurer la température de l'eau, son PH, sa conductivité, son potentiel d'oxydoréduction (représentatif de la concentration en agent bactéricide dans l'eau, tel que le chlore, le brome ou l'oxygène actif) dans l'eau. La surveillance de la qualité de l'eau permet de déclencher un traitement approprié de l'eau. La pratique courante consiste à introduire des produits d'entretien adaptés, en quantité adaptée, à actionner une pompe d'oxygénation de l'eau ou bien un dispositif de filtration de l'eau.

Du document WO2007/02530, on connait un système de surveillance flottant embarquant un capteur apte à mesurer une grandeur représentative de la qualité de l'eau. Toutefois, le taux de fausses alarmes de ce type de dispositif est important. La mesure de la qualité de l'eau en un point du bassin n'est pas forcément représentative de la qualité de l'eau du bassin dans sa globalité. Le document WO2004/019295 divulgue un système de surveillance d'un bassin d'eau comprenant un robot de nettoyage submersible doté de capacités de propulsion autonomes. Ce robot incorpore des capteurs qui mesurent les conditions de l'eau du bassin par rapport à un état de référence. Le robot est aussi muni de moyens de communication avec un deuxième élément situé à l'extérieur du bassin. Le document US2008/106422 divulgue un système submergé adapté pour détecter des evenements de chute dans un bassin d'eau et pour mesurer la qualité de l'eau du bassin. On connait un système de surveillance d'immersion installé sur le rebord du bassin et intégrant une sonde plongeant dans l'eau apte à détecter l'onde acoustique générée par la chute d'un corps dans le bassin. Toutefois, le nombre de fausses alarmes (détection d'immersion sans évènement d'immersion ou absence de détection d'immersion alors qu'un évènement d'immersion a eu lieu) est important. Par exemple, compte tenu de la force du vent, l'amortissement des remous provoqués par la chute d'un corps peut prendre un certain temps, voir ne pas se produire lorsque la vitesse du vent est supérieure à 36km/h. Ce phénomène détériore la sensibilité du système.

Le but de la présente invention est de proposer des systèmes de surveillance de bassins qui soient fiables, c'est-à-dire qui présentent un taux de fausses alarmes faible.

A cet effet, l'invention a pour objet un procédé de surveillance d'un bassin en accord avec la revendication 1. L'invention a également pour objet un procédé de surveillance d'un bassin contenant de l'eau à partir d'au moins un premier élément comprenant au moins un robot submersible doté de capacités de propulsion autonomes dans ledit bassin et étant doté de capacités de communication en position immergée avec au moins un deuxième élément permettant de communiquer avec ledit deuxième élément, ledit premier élément comprenant une pluralité de robots submersibles et/ou ledit deuxième élément étant un robot submersible, des capteurs aptes à produire des mesures d'une grandeur représentative d'au moins une perturbation dudit bassin par rapport à un état de référence étant embarqués à bord desdits robots submersibles, ledit procédé comprenant une étape de production de mesures d'une grandeur représentative d'au moins une perturbation dudit bassin par rapport à un état de référence, lesdites mesures étant issues desdits capteurs, une étape de communication dudit premier élément en position immergée avec au moins un deuxième élément, une étape de traitement des sorties desdits capteurs, pour déclencher moins une action d'information sur la perturbation du bassin par rapport à un état de référence au moyen de moyens d'information.

Avantageusement, le deuxième élément est un robot submersible doté de capacités de propulsion autonomes dans ledit bassin, sur lequel est embarqué au moins un capteur apte à produire au moins une mesure d'une grandeur représentative d'au moins une perturbation dudit bassin par rapport à un état de référence, ledit système étant en outre configuré pour traiter dans les moyens de traitement la sortie dudit au moins un capteur embarqué sur ledit deuxième élément.

Avantageusement, ledit au moins un capteur est en outre apte à détecter l'intrusion d'un individu dans le bassin et dans lequel, le premier élément comprend une pluralité de robots submersibles et/ou le deuxième élément est constitué par un robot submersible. Le procédé comprend :
- une étape de détection d'une intrusion d'un corps dans l'eau lors de laquelle ledit au moins un capteur produit, lorsqu'il détecte l'intrusion d'un individu dans l'eau, un premier signal d'alerte A1 ainsi qu'un premier signal de probabilité PA1, PB1, PC1 représentatif de la probabilité avec laquelle l'intrusion est susceptible de s'être réellement produite,
- et, lorsqu'un premier signal d'alerte A1 est produit par un premier robot submersible, une étape de confirmation lors de laquelle on vérifie, au moins à partir d'un signal de probabilité issu d'au moins un deuxième robot submersible, si l'intrusion d'un individu est confirmée par au moins un deuxième robot submersible.

Avantageusement, lorsque l'intrusion est confirmée, une étape de pilotage de moyens d'information de façon à informer un individu de la survenue d'une chute d'un individu dans l'eau du bassin.

Avantageusement, l'étape de confirmation comprend :
- une première étape de comparaison du nombre de robots submersibles N ayant produit un premier signal d'alerte A1 avec un nombre seuil prédéterminé,
- et, lorsque le nombre de robots submersibles N ayant produit un premier signal d'alerte A1 est au moins égal au nombre seuil prédéterminé, une deuxième étape de comparaison de premiers signaux de probabilités PA1, PB1, PC1, associés aux signaux d'alerte A1 respectifs, avec un premier seuil de probabilité prédéterminé S1.

Avantageusement, l'étape de confirmation comprend, lorsqu'au moins un signal de probabilité est inférieur au premier seuil prédéterminé, respectivement lorsque le nombre de robots submersibles ayant produit un premier signal d'alerte A1 est inférieur au nombre seuil prédéterminé,
- une étape d'identification d'un robot submersible principal correspondant au robot submersible dont est issu le premier signal de probabilité dont la valeur est la plus élevée,
- une étape d'actionnement des moyens d'entraînement d'au moins un autre robot submersible, correspondant à un robot submersible autre que le robot submersible principal, de façon qu'il se rapproche du robot submersible principal,
- une deuxième étape de détection lors de laquelle ledit capteur embarqué à bord dudit au moins un autre robot submersible produit un signal de probabilité représentatif de la probabilité avec laquelle l'intrusion est susceptible de s'être réellement produite,
- une troisième étape de comparaison lors de laquelle on compare ledit au moins un deuxième signal de probabilité avec un deuxième seuil de probabilité.

Avantageusement, l'évènement d'intrusion étant confirmé lorsqu'au moins un deuxième signal de probabilité est supérieur au deuxième seuil prédéterminé.

Avantageusement, le nombre seuil est égal à trois.

D'autres caractéristiques et avantages de l'invention apparaîtront à la lecture de la description détaillée qui suit, faite à titre d'exemple non limitatif et en référence aux dessins annexés dans lesquels :
- la figure 1 représente schématiquement un système selon un premier exemple,
- la figure 2 représente schématiquement un système selon un deuxième exemple,
- la figure 3 représente les étapes principales du procédé selon l'invention,
- la figure 4 représente les étapes détaillées d'un mode de réalisation préféré du procédé selon l'invention,
- la figure 5 représente un exemple de disposition de capteurs embarqués sur un robot submersible.

D'une figure à l'autre, les mêmes éléments sont repérés par les mêmes références.

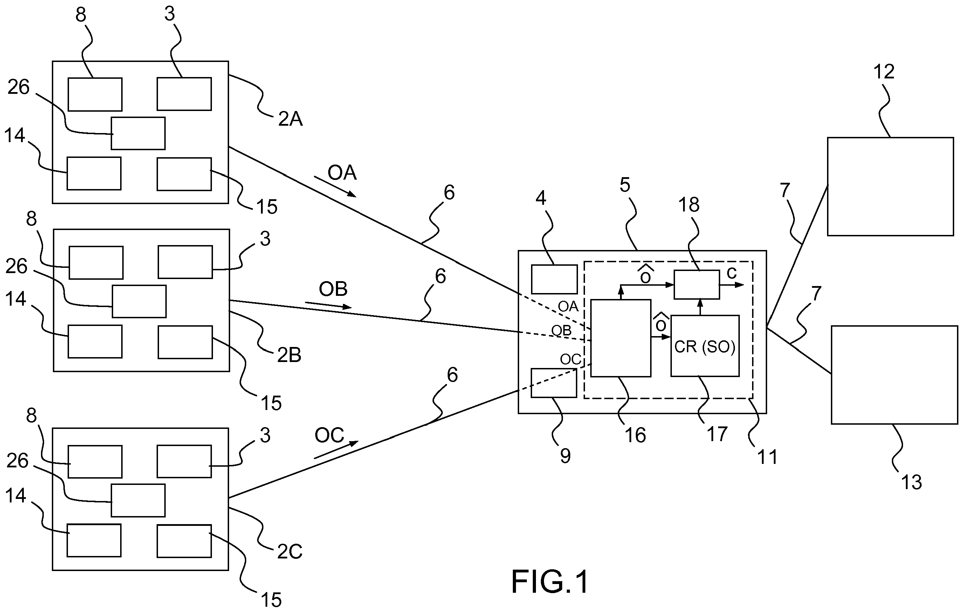

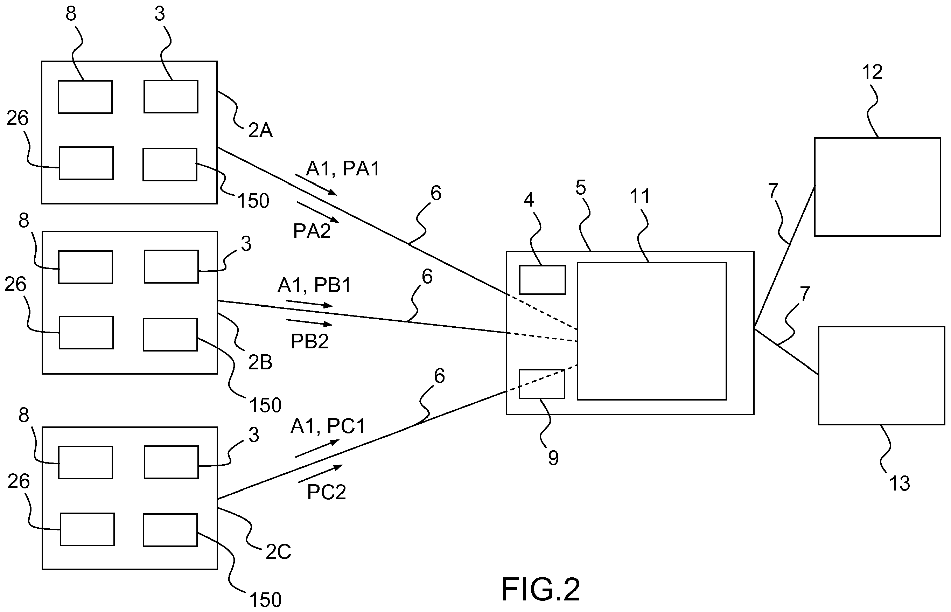

Sur les figures 1 et 2, on a représenté schématiquement deux exemples différents d'un système de surveillance d'un bassin. Dans ces exemples le liquide contenu dans le bassin est de l'eau. Tout ce qui est décrit dans la suite du texte pour l'eau est aussi valable pour tout autre liquide transparent.

Le système selon le premier exemple représenté sur la figure 1, est un système de surveillance de la qualité de l'eau stockée dans le bassin.

Le système selon le deuxième exemple représenté sur la figure 2, est un système de surveillance de la survenue d'une intrusion d'un individu dans l'eau stockée dans le bassin. Nous décrirons tout d'abord les éléments communs aux deux exemples.

Les systèmes de surveillance selon les deux exemples représentés comprennent un premier élément constitué de 3 robots submersibles 2A, 2B, 2C dotés de capacités de propulsion autonomes 3. Les robots submersibles sont aptes à se déplacer librement dans l'eau.

Les moyens de propulsion autonomes 3 sont configurés pour communiquer des mouvements aux robots submersibles 2A, 2B, 2C dans les trois dimensions dans l'eau.

Avantageusement, les robots submersibles sont des robots sous-marins. Ils sont configurés pour être submergés et naviguer en profondeur.

Les moyens de propulsion 3 sont, par exemple, agencés pour communiquer, aux robots submersibles, des mouvements similaires à ceux des poissons.

Les systèmes comprennent en outre des moyens d'actionnement 4 des moyens de propulsion 3. Dans les modes de réalisation représentés, les moyens d'actionnement 4 sont déportés et installés sur un deuxième élément constitué par une unité centrale de traitement 5. L'unité centrale de traitement 5 est configurée pour flotter sur l'eau du bassin.

En variante, les moyens d'actionnement 4 sont embarqués à bord des robots submersibles 2A, 2B, 2C. Les robots submersibles sont alors aptes à se déplacer de façon autonome dans le milieu liquide. Les moyens d'actionnement 4 peuvent également être embarqués à bord d'un des robots submersibles qui est alors le robot maître.

La centrale flottante 5 embarque différents équipements qui seront décrits ultérieurement. Cet agencement permet de supprimer le temps d'installation des équipements embarqués à bord de la centrale flottante sur la structure du bassin.

Le premier élément, constitué de trois robots submersibles respectifs 2A, 2B, 2C, est doté de capacités de communication en position immergée et avantageusement submergée avec la centrale flottante 5 et/ou avec les autres robots submersibles 2A, 2B, 2C respectifs.

Ces capacités de communication permettent à la centrale flottante 5 et aux robots submersibles 2A, 2B, 2C de communiquer via des moyens de liaison sans fil 6. Elles permettent à la centrale 5 de commander à distance les moyens de propulsion 3.

Les moyens de liaison sans fil sont, par exemple, un émetteur et un récepteur de type sismique, acoustique, optique (infrarouge ou visible) ou de tout autre type apte à transmettre des données dans un milieu liquide, en particulier dans l'eau. La transmission de données au moyen d'ondes acoustiques très basse fréquence ou d'ondes électromagnétiques infrarouges ou visibles est particulièrement adaptée à l'invention. Les ondes très basse fréquence appelées VLF, en référence, à l'expression anglo-saxonne « very low frequency » dont la fréquence est comprise entre 3 et 30 HZ peuvent être transmises pratiquement sans atténuation dans un milieu liquide. A l'inverse, les ondes radio fréquence RF sont presque entièrement absorbées par le milieu liquide où la propagation de ces ondes est extrêmement limitée.

Les robots submersibles 2A, 2B, 2C comprennent des moyens d'accumulation d'énergie 8 aptes à alimenter en énergie électrique les équipements embarqués à bord desdits robots 2.

Des moyens d'alimentation en énergie électrique 9 destinés à coopérer avec les moyens d'accumulation d'énergie 8 pour assurer leur recharge, par exemple par induction, sont embarqués à bord de l'unité centrale 5.

Avantageusement, les moyens d'actionnement 4 sont configurés de sorte à diriger les robots submersibles 2A, 2B, 2C vers l'unité centrale de traitement 5 pour venir faire coopérer les moyens d'alimentation 9 avec les moyens d'accumulation 8 de sorte à assurer la recharge de ces derniers, lorsque l'énergie électrique stockée dans les moyens d'accumulation est inférieure à un seuil d'énergie prédéterminé. Le premier élément est constitué, sur les réalisations des figures 1 et 2, par les trois robots submersibles 2A, 2B, 2C dotés de capacités de propulsion autonomes et sur lesquels sont embarqués des capteurs 15, 150. Les capteurs 15, 150 sont aptes à produire des mesures d'une grandeur représentative d'au moins une perturbation du bassin par rapport à un état de référence.

Avantageusement, les robots comprennent des capacités de localisation en trois dimensions non représentées. Ces capacités de localisation comprennent avantageusement des moyens pour localiser le robot en trois dimensions. Il peut, par exemple, s'agir d'une centrale inertielle.

En variante, les capacités de localisation d'un robot submersible comprennent des capacités de communication avec au moins un robot submersible et/ou l'unité centrale de traitement 5. Les moyens de traitement 11 (décrits ci-dessous) sont alors configurés pour positionner les robots à partir de signaux de communication issus de capacité de communication, par exemple, par triangulation.

Le système est configuré pour traiter dans des moyens de traitement 11 les sorties des capteurs 15, 150 et éventuellement des capacités de localisation et déclencher au moins une action.

Les actions déclenchées peuvent, par exemple, être des actions d'information d'un individu de la perturbation du bassin par rapport à un état de référence.

Les moyens de traitement 11 sont aptes à générer des commandes pour piloter des moyens d'information 12, 13, 14 en utilisant des mesures des grandeurs représentatives d'au moins une perturbation du bassin par rapport à un état de référence et éventuellement des capacités de localisation.

Les moyens de traitement 11 sont déportés. Ils sont installés à bord de l'unité centrale 5. En variante, les moyens de traitement sont embarqués à bord d'un robot submersible.

Les moyens d'information comprennent, dans les deux modes de réalisation, des moyens d'affichage 12 disposés à l'extérieur du bassin. Les moyens d'affichage sont, en variante, installés sur l'unité centrale 5.

Les moyens d'information comprennent en outre un dispositif d'alarme 13. Ce dispositif est, par exemple, apte à produire une alarme visuelle ou sonore. Le dispositif d'alarme peut être déporté. Il peut être installé à l'extérieur du bassin de loisir.

Le système comprend en outre des moyens de communication 7 permettant l'échange de données entre les moyens de traitement 11 et les moyens d'information respectifs 12, 13, 14. Ces moyens de communication sont, par exemple, des liaisons sans fil.

Nous allons maintenant décrire plus précisément le système de surveillance de la qualité de l'eau représenté sur la figure 1.

Dans cet exemple, par perturbation du bassin on entend une perturbation créée par une modification d'un paramètre physicochimique de l'eau, au-delà d'un seuil de référence. On parle donc de perturbation de l'eau contenue dans le bassin.

Les capteurs 15 sont aptes à produire des mesures d'un paramètre physico-chimique de l'eau.

Ces paramètres physicochimiques sont des grandeurs représentatives de la qualité de l'eau dans laquelle baigne le robot submersible.

Les capteurs 15 sont, par exemple, aptes à produire des mesures OA, OB, OC du potentiel d'oxydoréduction du milieu liquide dans lequel baigne le robot submersible. Le potentiel d'oxydoréduction est représentatif de la concentration du désinfectant (chlore, brome, oxygène actif) dans l'eau.

En variante, le capteur est un thermomètre, ou bien un capteur apte à mesurer le PH ou la conductivité du milieu aqueux dans lequel baigne le capteur.

En variante, les robots submersibles embarquent une pluralité de capteurs 15, susceptibles de mesurer différentes grandeurs physicochimiques de la qualité de l'eau.

En variante, le système comprend 1, 2 ou plus de 3 robots submersibles.

Les moyens d'information comprennent, par exemple, des moyens d'information embarqués 14 aptes à modifier l'aspect visuel des robots submersibles à partir d'une commande provenant des moyens de traitement 11.

Les moyens d'information embarqués 14 comprennent des diodes électroluminescentes.

Les diodes électroluminescentes sont agencées de façon à produire un éclairage visible depuis l'extérieur des robots submersibles. Elles sont, par exemple, installées à la surface des robots submersibles. Elles peuvent aussi être installées à l'intérieur d'un boitier délimitant l'engin submersible, ledit boitier étant transparent au rayonnement électromagnétique visible.

Ces moyens 14, non représentés sur la figure 2, pourraient être installés sur les robots submersibles du système selon le deuxième mode de réalisation.

Les moyens de traitement 11 comprennent des moyens d'estimation 16 aptes à produire, à partir d'au moins une mesure d'un paramètre physico-chimique de l'eau, une estimation de la valeur dudit paramètre physico-chimique.

Les moyens de traitement 11 comprennent en outre des moyens 17 pour évaluer la qualité de l'eau aptes à vérifier si l'eau respecte un critère de qualité prédéterminé CR en comparant au moins une estimation de la valeur du paramètre physico-chimique avec un seuil de référence relatif au paramètre considéré.

Dans le cas où plusieurs capteurs sont embarqués à bord d'un même robot les moyens 17 reçoivent des estimations de plusieurs grandeurs. Le critère de qualité de l'eau peut être un critère de qualité composite. Les moyens 17 comparent alors les évaluations des valeurs de plusieurs paramètres physico-chimiques mesurés avec des seuils prédéterminés respectifs. Cela revient à évaluer la qualité de l'eau à partir de mesures de plusieurs grandeurs. Cela permet d'améliorer la fiabilité du dispositif.

Dans l'exemple représenté sur la figure 1, l'action générée par le système est une action d'information d'un individu.

Les moyens de traitement 11 comprennent en outre des moyens 18 pour générer une commande de pilotage C des moyens d'information 12, 13, 14 à partir d'au moins une estimation de la valeur d'au moins une paramètre physico-chimique représentatif de la qualité de l'eau ou bien à partir de l'évaluation de la qualité de l'eau réalisée par les moyens d'évaluation 17.

Selon un mode de fonctionnement du dispositif les capteurs 15 embarqués à bord des robots submersibles réalisent des mesures OA, OB, OC du potentiel d'oxydoréduction. Ce qui est dit après est également valable lorsqu'on mesure un autre type de paramètre physicochimique.

Ces mesures sont réalisées régulièrement. Ces mesures sont transmises régulièrement aux moyens de traitement 11 au moyen des premiers moyens de communication.

L'estimation Ô de la valeur du potentiel d'oxydoréduction est calculée à partir de plusieurs mesures de ce potentiel réalisées par un unique capteur. Il s'agit, par exemple, d'une moyenne d'une pluralité de mesures OA réalisées par un capteur 15 à différents instants.

Les moyens d'évaluation 17 reçoivent ensuite une estimation Ô du potentiel d'oxydoréduction. Ils comparent cette estimation Ô à un seuil d'oxydoréduction SO prédéterminé. Si le potentiel est supérieure, respectivement inférieur, à ce seuil, cela signifie que la concentration en agent désinfectant est insuffisante, respectivement suffisante. Les moyens 18 pour générer une commande génèrent alors une commande pour allumer une diode émettant de la lumière rouge, respectivement verte. Le propriétaire d'une piscine, voyant l'engin submersible émettre une lumière rouge sait alors qu'il doit verser du désinfectant dans la piscine.

Les moyens 18 peuvent également générer une commande de déclenchement d'une alarme ou une commande d'affichage.

En estimant le potentiel d'oxydoréduction à partir de plusieurs mesures réalisées à des instants différents, on s'assure que l'estimation du potentiel d'oxydoréduction est précise. Le système de surveillance est alors fiable. Autrement dit, le nombre de fausses alarmes de ce système est faible. Un exploitant ou un propriétaire peut se fier à la couleur des robots submersibles pour savoir quand alimenter l'eau du bassin en désinfectant.

Avantageusement, les robots submersibles sont configurés pour se déplacer en permanence. Le calcul du potentiel d'oxydoréduction est alors réalisé à partir de mesures prises en différents points de la piscine, ce qui améliore la précision de l'estimation du potentiel et la fiabilité du système selon l'invention.

En variante, l'estimation Ô de la valeur du potentiel d'oxydoréduction est calculée à partir de mesures de ce potentiel réalisées par différents capteurs installés sur des robots submersibles différents (le système comprend alors au moins deux robots submersibles). Elle est, par exemple, calculée en réalisant la moyenne des mesures issues des différents capteurs.

Les mesures utilisées pour estimer le potentiel d'oxydoréduction sont réalisées à différents points du bassin puisque les différents robots submersibles occupent nécessairement des positions différentes (la précision est alors directement liée au nombre de robots utilisés).

La fiabilité du dispositif est encore améliorée lorsque les robots submersibles sont configurés pour être submergés. On garantit alors que les mesures sont réalisées en profondeur. Or, il a été prouvé que les mesures réalisées en profondeur sont plus représentatives de la qualité de l'eau que celles qui sont réalisées en surface.

La figure 2 représente un système selon un deuxième exemple. Ce système est un système de détection d'intrusion apte à détecter l'intrusion d'un corps dans l'eau du bassin.

Les capteurs 150 aptes à produire des mesures d'une grandeur représentative d'une perturbation du bassin et plus précisément du liquide contenu dans le bassin, par rapport à un état de référence sont embarqués à bord de chacun des robots submersibles 2A, 2B, 2C. Dans cet exemple la perturbation est une intrusion d'un individu dans le bassin.

Les capteurs 150 sont, par exemple, aptes à détecter une intrusion d'un corps, par exemple d'un individu, dans le bassin.

Dans cet exemple les capteurs 150 sont aptes à détecter l'intrusion d'un individu dans le liquide contenu dans le bassin qui de l'eau dans l'exemple décrit.

Ils sont, par exemple, aptes à produire une mesure d'une grandeur qui varie lors d'une intrusion d'un individu dans le liquide contenu dans le bassin. L'intrusion est, par exemple, détectée si la grandeur subit une variation brutale, c'est-à-dire si la variation de la grandeur pendant un intervalle de temps prédéterminé est supérieure à un seuil prédéterminé. Les capteurs 150 sont, par exemple, aptes à mesurer la luminosité et à détecter une variation brutale de cette luminosité. Ils peuvent également être aptes à détecter une intrusion lorsque la luminosité passe en dessous d'un seuil prédéterminé.

Les capteurs 150 peuvent également être de type sonore, à savoir, aptes à détecter l'onde de choc créée par l'immersion puis la trace sonore des bulles.

Les capteurs 150 sont aptes à produire des signaux de probabilité représentatifs de la probabilité avec laquelle l'intrusion est susceptible de s'être produite. Les capteurs sont en outre aptes à produire des signaux d'alerte lorsqu'ils détectent une intrusion, c'est-à-dire un évènement d'immersion.

Par exemple, un signal d'alerte est produit lorsque la vitesse de la variation de la luminosité est supérieure à un seuil de détection prédéterminé et le signal de probabilité associé au signal d'alerte est proportionnel à la vitesse de variation de la probabilité.

Les signaux d'alerte et de probabilité sont envoyés au deuxième élément qui est, sur la figure 2, une unité centrale flottante 5 et qui embarque les moyens de traitement 11.

On va maintenant décrire un procédé de mise en oeuvre du dispositif selon l'invention.

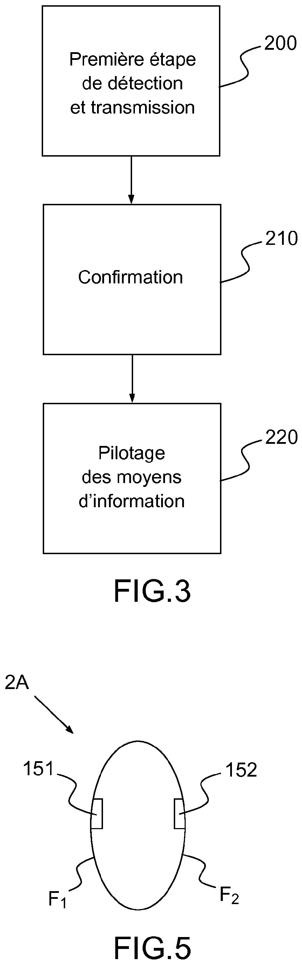

Les étapes majeures du procédé sont représentées sur la figure 3.

La mise en oeuvre de ce procédé nécessite que le système selon le deuxième mode de réalisation de l'invention comprenne plusieurs robots submersibles embarquant chacun au moins un capteur 150.

Le procédé comprend une première étape de détection 200 de l'intrusion d'un individu dans l'eau du bassin de loisir lors de laquelle les capteurs 150 produisent, lorsqu'ils détectent la chute d'un individu dans l'eau, un premier signal d'alerte A1 ainsi qu'un premier signal de probabilité PA1, PB1, PC1 représentatif de la probabilité avec laquelle l'intrusion d'un individu dans l'eau du bassin est susceptible de s'être réellement produite. Cette étape comprend en outre une étape de transmission des premiers signaux aux moyens de traitement 11. Ici les données sont transmises au deuxième élément qui est l'unité centrale de traitement 5.

Lorsqu'au moins un premier signal d'alerte A1 est envoyé aux moyens de traitement 11 par un premier robot submersible, le procédé comprend une étape de confirmation 210 consistant à vérifier, au moins à partir d'un premier signal de probabilité issu d'un ou plusieurs deuxième(s) robot(s) submersible(s) différentes du premier robot submersible, si la chute d'un individu est confirmée par au moins un deuxième robot submersible.

Cette étape de confirmation permet d'améliorer la fiabilité du dispositif selon l'invention par rapport à un dispositif qui comprendrait un unique détecteur. Lorsqu'un évènement d'immersion est confirmé par le dispositif, la probabilité que l'immersion se soit réellement produite est importante. L'étape de confirmation permet de discriminer les fausses détections.

Si l'intrusion est confirmée, le procédé comprend une étape de pilotage 220 des moyens d'information au moyen des moyens de traitement 11 de façon à informer un individu de la survenue d'une chute d'un individu dans l'eau du bassin. Il s'agit, par exemple, de piloter le dispositif d'alarme de façon à émettre une alarme sonore et/ou visuelle ou bien de piloter un dispositif d'affichage de façon à afficher une information.

Selon un mode de réalisation préféré, le dispositif selon le deuxième mode de réalisation comprend au moins trois robots submersibles.

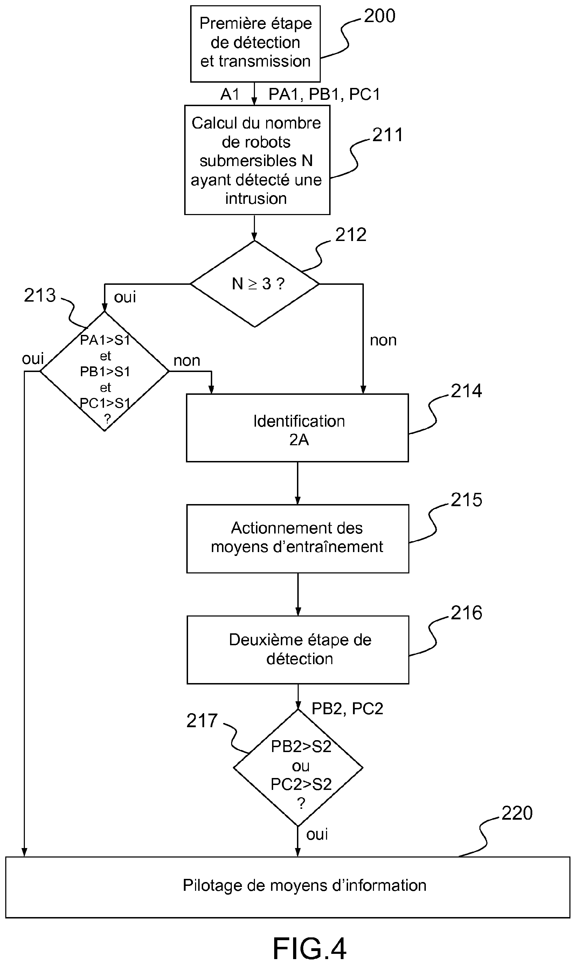

Sur la figure 4, on a représenté schématiquement les étapes du procédé dans ce cas.

L'étape de confirmation 210 comprend une étape de calcul 211 du nombre de robots submersibles N ayant transmis un premier signal d'alerte aux moyens de traitement 11 ainsi qu'une première étape de comparaison 212 du nombre d'engins submersibles N à un nombre seuil prédéterminé. Ce nombre seuil est égal à 3 dans l'exemple représenté sur la figure 4.

Avantageusement, cette étape consiste à comptabiliser le nombre de robots submersibles ayant transmis un premier signal d'alerte A1 aux moyens de traitement 11 dans une fenêtre de temps prédéterminée.

Si au moins trois robots submersibles ont détecté une intrusion, alors, l'étape de confirmation comprend une deuxième étape de comparaison 213 des premiers signaux de probabilité PA1, PB1, PC1 accompagnant les premiers signaux d'alertes A1, avec un premier seuil de probabilité prédéterminé S1.

Si, tous les premiers signaux de probabilité PA1, PB1, PC1, associés aux premiers signaux d'alerte A1 respectifs, sont supérieurs au premier seuil prédéterminé S1, la survenue de l'événement d'intrusion est confirmée.

Sinon, l'étape de confirmation 210 comprend en outre avantageusement (mais pas obligatoirement), une étape d'identification 214 d'un robot submersible principal correspondant au robot submersible 2A dont est issu le premier signal de probabilité dont la valeur est la plus élevée.

Par ailleurs, l'étape de confirmation 210 comprend une étape d'actionnement 215 des moyens d'entraînement des autres robots submersibles 2B, 2C correspondant aux robots submersibles autres que le robot submersible principal 2A, de façon qu'ils se rapprochent du robot submersible principal 2A. Cette étape est réalisée par les moyens d'actionnement à partir des mesures des positions des différents robots submersibles issues des capacités de localisation.

L'étape de confirmation comprend une deuxième étape de détection 216 lors de laquelle les moyens de détection 150 des autres robots submersibles 2B, 2C produisent des deuxièmes signaux de probabilité PB2, PC2 et les transmettent aux moyens de traitement 11 via les moyens de communication sans fil 6.

Cette étape est suivie d'une troisième étape de comparaison 217 des deuxièmes signaux de probabilité PB2, PC2 issus des autres robots submersibles avec un deuxième seuil de probabilité prédéterminé S2. L'évènement d'immersion est avantageusement confirmé si au moins un deuxième signal de probabilité PB2, PC2 issu d'au moins un autre robot submersible 2B, 2C est supérieur au deuxième seuil prédéterminé S2.

En variante, lors de la phase d'actionnement 215, on dirige seulement une partie des autres robots submersibles en direction du robot submersible principal. La fiabilité de la détection est d'autant plus importante que le nombre de robots submersibles dirigés vers le robot principal est important.

Si les moyens de traitement 11 reçoivent des premiers signaux d'alerte depuis moins de 3 robots submersibles, l'étape de confirmation 210 ne comprend pas la première étape de comparaison 213.

Etant donné que le rapprochement des autres robots submersibles 2B, 2C du robot submersible principal 2A n'est pas instantané, le deuxième seuil de probabilité S2 est avantageusement inférieur au premier seuil. En effet, les conséquences de l'intrusion d'un corps dans le bassin s'atténuent dans le temps. Par exemple, dans le cas de la mesure d'ondes acoustiques, l'onde de choc produit par l'évènement d'immersion a le temps de s'atténuer.

En variante le premier seuil de probabilité S1 est égal au deuxième seuil de probabilité S2. Ce mode de réalisation fait, par exemple, sens lorsque les moyens de détection sont de type optique et que les signaux de probabilité sont inversement proportionnels à la luminosité.

De façon à mettre en oeuvre l'étape d'actionnement 215, on utilise avantageusement les capacités de localisation des robots submersibles.

Avantageusement, comme représenté sur la figure 5, un capteur 150 embarqué à bord d'un robot submersible 2A comprend deux capteurs élémentaires 151, 152 disposés de façon à être espacés l'un de l'autre dans un plan horizontal lorsque l'engin submersible est immergé. Le système comprend avantageusement des moyens pour détecter une zone d'immersion dans laquelle l'immersion est supposée s'être produite à partir des signaux issus de deux capteurs élémentaires.

Par exemple, les deux capteurs élémentaires 151, 152 sont agencés sur les flancs respectifs F1, F2 de l'engin submersible 2A. Avantageusement, le robot submersible 2A présente une forme oblongue, dans un plan horizontal, lorsque l'engin submersible est immergé.

Le système selon l'invention présente une bonne fiabilité. En effet, les capteurs aptes à détecter une intrusion présentent une meilleure sensibilité lorsqu'ils sont placés sous l'eau que lorsqu'ils sont placés en surface.

De façon à mettre en oeuvre le procédé précédemment décrit, les moyens de traitement comprennent les moyens suivants (qui ne sont pas représentés sur la figure 2) :
- des moyens de calcul du nombre d'engins submersibles N ayant détecté une intrusion,
- des moyens pour comparer le nombre d'engins submersibles au nombre seuil,
- des moyens pour comparer des signaux de probabilité à un seuil prédéterminé de manière à pouvoir mettre en oeuvre les deux étapes de comparaison 213, 217,
- des moyens pour identifier l'engin submersible ayant transmis aux moyens de traitement le signal de probabilité le plus important.

Ces moyens sont avantageusement des modules de calcul.

Le système est facile à installer. Il ne nécessite pas d'opérations d'installation sur le bassin. Les capteurs sont notamment montés sur un robot submersible à l'extérieur du bassin. Le robot est ensuite immergé dans l'eau du bassin. La maintenance du dispositif de surveillance est en outre facilitée.

Dans des variantes de réalisation de l'invention, le premier élément est constitué d'un ou plusieurs robots submersibles 2A, 2B, 2C tels que décrits précédemment. Le deuxième élément est aussi constitué d'un robot submersible 2A, 2B, 2C tel que décrit précédemment. On parle alors de robots maître-esclaves. Le système est alors en outre configuré pour traiter, dans les moyens de traitement les sorties du (ou des) capteurs 15, 150 embarqués à bord du deuxième élément et éventuellement des capacités de localisation dudit deuxième élément.

Les moyens de traitement 11 peuvent être embarqués à bord du robot maître (deuxième élément). Ils peuvent également être déportés et dotés de capacités de communication avec le deuxième élément (robot maître). Le robot maître reçoit alors les sorties des capteurs 15, 150 et éventuellement des capacités de localisation des robots esclaves et également du robot maître et les transmet aux moyens de traitement. Autrement dit, dans ce cas, le système comprend un ensemble de robots comprenant un premier ensemble de robots correspondant aux robots du premier élément et un robot supplémentaire correspondant au deuxième élément. Ce robot supplémentaire est le robot maitre et le(s) robot(s) du premier élément est (ou sont) des robot(s) esclaves.

Les moyens d'actionnement 3 peuvent être embarqués à bord du robot maître.

Par ailleurs, lorsque les systèmes selon les premier et deuxième modes de réalisation comprennent plusieurs robots submersibles, ceux-ci peuvent être répartis entre le premier et le deuxième élément. Le premier élément peut être constitué d'un ou plusieurs robots submersibles tels que décrits précédemment et le deuxième élément être constitué d'un robot submersible tel que décrit précédemment.

## Revendications

1. Procédé de surveillance d'un bassin contenant de l'eau à partir d'au moins un premier élément comprenant au moins un robot submersible (2A, 2B, 2C) doté de capacités de propulsion autonomes dans ledit bassin et étant doté de capacités de communication en position immergée avec au moins un deuxième élément (2A, 2B, 2C, 5) permettant de communiquer avec ledit deuxième élément, ledit premier élément comprenant une pluralité de robots submersibles et/ou ledit deuxième élément étant un robot submersible, des capteurs (15, 150) aptes à produire des mesures d'une grandeur représentative d'au moins une perturbation dudit bassin par rapport à un état de référence étant embarqués à bord desdits robots submersibles, ledit procédé comprenant une étape de production de mesures d'une grandeur représentative d'au moins une perturbation dudit bassin par rapport à un état de référence, lesdites mesures étant issues desdits capteurs (15), une étape de communication dudit premier élément en position immergée avec au moins un deuxième élément (2A, 2B, 2C, 5), une étape de traitement des sorties desdits capteurs (15, 150), pour déclencher moins une action d'information sur la perturbation du bassin par rapport à un état de référence au moyen de moyens d'information, le procédé comprenant en outre, une étape de détection (200) d'une intrusion d'un corps dans l'eau lors de laquelle ledit au moins un capteur (150) produit, lorsqu'il détecte l'intrusion d'un individu dans l'eau, un premier signal d'alerte A1 ainsi qu'un premier signal de probabilité PA1, PB1, PC1 représentatif de la probabilité avec laquelle l'intrusion est susceptible de s'être réellement produite,
- et, lorsqu'un premier signal d'alerte A1 est produit par un premier robot submersible, une étape de confirmation (210) lors de laquelle on vérifie, au moins à partir d'un signal de probabilité issu d'au moins un deuxième robot submersible, si l'intrusion d'un individu est confirmée par au moins un deuxième robot submersible.

2. Procédé selon la revendication précédente, comprenant, lorsque l'intrusion est confirmée, une étape de pilotage (220) de moyens d'information de façon à informer un individu de la survenue d'une chute d'un individu dans l'eau du bassin.

3. Procédé de surveillance selon l'une quelconque des revendications précédentes, dans lequel l'étape de confirmation (210) comprend :
- une première étape de comparaison (212) du nombre de robots submersibles N ayant produit un premier signal d'alerte A1 avec un nombre seuil prédéterminé,
- et, lorsque le nombre de robots submersibles N ayant produit un premier signal d'alerte A1 est au moins égal au nombre seuil prédéterminé, une deuxième étape de comparaison (213) de premiers signaux de probabilités PA1, PB1, PC1, associés aux signaux d'alerte A1 respectifs, avec un premier seuil de probabilité prédéterminé S1.

4. Procédé de surveillance selon la revendication 3, dans lequel l'étape de confirmation (210) comprend, lorsqu'au moins un signal de probabilité est inférieur au premier seuil prédéterminé, respectivement lorsque le nombre de robots submersibles ayant produit un premier signal d'alerte A1 est inférieur au nombre seuil prédéterminé,
- une étape d'identification (214) d'un robot submersible principal correspondant au robot submersible dont est issu le premier signal de probabilité dont la valeur est la plus élevée,
- une étape d'actionnement (215) des moyens d'entraînement (4) d'au moins un autre robot submersible, correspondant à un robot submersible autre que le robot submersible principal, de façon qu'il se rapproche du robot submersible principal,
- une deuxième étape de détection (216) lors de laquelle ledit capteur (150) embarqué à bord dudit au moins un autre robot submersible produit un signal de probabilité représentatif de la probabilité avec laquelle l'intrusion est susceptible de s'être réellement produite,
- une troisième étape de comparaison (217) lors de laquelle on compare ledit au moins un deuxième signal de probabilité avec un deuxième seuil de probabilité.

5. Procédé selon la revendication 4, dans lequel l'évènement d'intrusion étant confirmé lorsqu'au moins un deuxième signal de probabilité est supérieur au deuxième seuil prédéterminé.

6. Procédé selon l'une quelconque des revendications 4 à 5, dans lequel le nombre seuil est égal à trois.

## Patentansprüche

1. Überwachungsverfahren eines Beckens, das Wasser enthält, anhand von mindestens einem ersten Element, das mindestens einen Tauchroboter (2A, 2B, 2C), der über autonome Antriebskapazitäten in dem Becken verfügt und in getauchter Position über Kommunikationskapazitäten mit mindestens einem zweiten Element (2A, 2B, 2C, 5) verfügt, das eine Kommunikation mit dem zweiten Element ermöglicht, wobei das erste Element eine Vielzahl von Tauchrobotern umfasst und/oder das zweite Element ein Tauchroboter ist, über Geber (15, 150), die in der Lage sind, Messungen einer Größe zu erzeugen, die mindestens eine Störung des Beckens in Bezug auf einen Referenzzustand darstellt, und die an Bord der Tauchroboter sind, das Verfahren umfassend einen Erzeugungsschritt von Messungen einer Größe, die mindestens eine Störung des Beckens in Bezug auf einen Referenzzustand darstellt, wobei die Messungen von den Gebern (15) ausgegeben werden, einen Kommunikationsschritt des ersten Elements in getauchter Position mit mindestens einem zweiten Element (2A, 2B, 2C, 5), einen Behandlungsschritt der Ausgänge der Geber (15, 150), um mindestens eine Informationsaktion über die Störung des Beckens in Bezug auf einen Referenzzustand auszulösen, das Verfahren umfassend außerdem einen Erfassungsschritt (200) eines Eindringens eines Körpers in das Wasser, wenn der mindestens eine Geber (150) bei Erfassen des Eindringens eines Individuums in das Wasser ein erster Warnsignal A1 sowie ein erstes Wahrscheinlichkeitssignal PA1, PB1, PC1, das die Wahrscheinlichkeit darstellt, mit der das Eindringen tatsächlich eingetreten ist, erzeugt,
- und wenn ein erstes Warnsignal A1 von einem ersten Tauchroboter erzeugt wird, einen ersten Bestätigungsschritt (210) bei dem anhand von mindestens einem Wahrscheinlichkeitssignal, das von mindestens einem zweiten Tauchroboter ausgegeben wird, überprüft wird, ob das Eindringen eines Individuums durch mindestens einen zweiten Tauchroboter bestätigt ist.

2. Verfahren nach dem vorherigen Anspruch, umfassend, wenn das Eindringen bestätigt ist, einen Steuerschritt (220) von Informationsmitteln, um ein Individuum über das Eintreten eines Sturzes eines Individuums in das Wasser des Beckens zu informieren.

3. Überwachungsverfahren nach einem der vorherigen Ansprüche, wobei der Bestätigungsschritt (210) umfasst:
- einen ersten Vergleichs schritt (212) der Anzahl von Tauchrobotern N, die ein erstes Warnsignal A1 erzeugt haben, mit einer vorbestimmten Schwellenanzahl,
- einen zweiten Vergleichsschritt (213) erster Wahrscheinlichkeitssignale PA1, PB1, PC1 die mit den jeweiligen Warnsignalen A1 assoziiert sind, mit einem ersten vorbestimmten Wahrscheinlichkeitsschwellenwert S1, wenn die Anzahl von Tauchrobotern N, die ein erstes Warnsignal A1 erzeugt haben, mindestens gleich der vorbestimmten Schwellenanzahl ist.

4. Überwachungsverfahren nach Anspruch 3, wobei der Bestätigungsschritt (210) umfasst, wenn mindestens ein Wahrscheinlichkeitssignal niedriger als der erste vorbestimmte Schwellenwert ist, bzw. wenn die Anzahl von Tauchrobotern, die ein erstes Warnsignal A1 erzeugt haben, niedriger als die vorbestimmte Schwellenanzahl ist,
einen Identifizierungsschritt (214) eines Haupt-Tauchroboters entsprechend dem Tauchroboter, von dem das erste Wahrscheinlichkeitssignal ausgegeben wird, dessen Wert am höchsten ist,
einen Betätigungsschritt (215) von Antriebsmitteln (4) von mindestens einem anderen Tauchroboter entsprechend einem anderen Tauchroboter als der Haupt-Tauchroboter, sodass er sich dem Haupt-Tauchroboter annähert,
einen zweiten Erfassungsschritt (216), bei dem der Geber (150) an Bord des mindestens einen anderen Tauchroboters ein Wahrscheinlichkeitssignal erzeugt, das die Wahrscheinlichkeit darstellt, mit der das Eindringen tatsächlich eingetreten ist,
einen dritten Vergleichsschritt (217), bei dem das mindestens eine zweite Wahrscheinlichkeitssignal mit einem zweiten Wahrscheinlichkeitsschwellenwert verglichen wird.

5. Verfahren nach Anspruch 4, wobei das Ereignis des Eindringens bestätigt ist, wenn mindestens ein zweites Wahrscheinlichkeitssignal höher als der zweite vorbestimmte Schwellenwert ist.

6. Verfahren nach einem der Ansprüche 4 bis 5, wobei die Schwellenanzahl gleich drei ist.

## Claims

1. A method for monitoring a pool containing water from at least one first element comprising at least one submersible robot (2A, 2B, 2C) provided with capabilities for autonomous propulsion in said pool and being provided with capabilities for communicating, in a submerged position, with at least one second element (2A, 2B, 2C, 5) that allow communication with said second element, said first element comprising a plurality of submersible robots and/or said second element being a submersible robot, sensors (15, 150) designed to produce measurements with a value that represents at least one disturbance of said pool compared to a reference state and being placed on board said submersible robots, said method comprising a step of producing measurements with a value that represents at least one disturbance of said pool compared to a reference state, said measurements originating from said sensors (15), a step of communicating with at least one second element (2A, 2B, 2C, 5) from said first element in a submerged position, a step of processing outputs from said sensors (15, 150), in order to trigger at least one action relating to information on the disturbance of the pool compared to a reference state by means of information means, said method further comprising a step (200) of detecting an intrusion of a body in the water, during which said at least one sensor (150) generates, when it detects the intrusion of an individual in the water, a first warning signal A1 and a first probability signal PA1, PB1, PC1 representing the probability with which the intrusion is likely to have actually occurred,
- and, when a first warning signal A1 is generated by a first submersible robot, a confirmation step (210), during which a check is carried out, at least on the basis of a probability signal originating from at least one second submersible robot, if the intrusion of an individual is confirmed by at least one second submersible robot.

2. The method according to the preceding claim, comprising, when the intrusion is confirmed, a step (220) of controlling information means so as to notify an individual that an individual has fallen into the water of the pool.

3. The monitoring method according to any one of the preceding claims, wherein said confirmation step (210) comprises:
- a first step (212) of comparing the number of submersible robots N that generated a first warning signal A1 to a predetermined threshold number; and
- a second step (213) of comparing first probability signals PA1, PB1, PC1, which are associated with the respective warning signals A1, to a first predetermined probability threshold S1 when the number of submersible robots N that generated a first warning signal A1 is at least equal to the predetermined threshold number.

4. The monitoring method according to claim 3, wherein said confirmation step (210) comprises, when at least one probability signal is lower than said first predetermined threshold, respectively when the number of submersible robots that generated a first warning signal A1 is lower than said predetermined threshold number:
- a step (214) of identifying a main submersible robot corresponding to the submersible robot from which the first probability signal with the highest value originated;
- a step (215) of actuating means (4) for driving at least one further submersible robot corresponding to a submersible robot other than the main submersible robot, so that it approaches the main submersible robot;
- a second detection step (216), during which said sensor (150) on board said at least one further submersible robot generates a probability signal that represents the probability with which the intrusion is likely to have actually occurred;
- a third comparison step (217), during which said at least one second probability signal is compared to a second probability threshold.

5. The method according to claim 4, wherein the intrusion event is confirmed when at least one second probability signal is higher than the second predetermined threshold.

6. The method according to any one of claims 4 to 5, wherein the threshold number is three.
